# EUROPEAN PATENT SPECIFICATION

(11) **EP 4 026 480 B1**
(45) Date of publication and mention of the grant of the patent: **13.05.2026**
(21) Application number: 21212701.3
(22) Date of filing: 07.12.2021
(51) Int. Cl.: A61B 3/00

(54) **HORIZONTAL AND VERTICAL ROTATION DRIVING DEVICE AND EYE-EXAMINING DEVICE HAVING THE SAME**
HORIZONTALE UND VERTIKALE ROTATIONSANTRIEBSVORRICHTUNG UND AUGENUNTERSUCHUNGSVORRICHTUNG UNTER VERWENDUNG DERSELBEN
DISPOSITIF D'ENTRAÎNEMENT À ROTATION HORIZONTALE ET VERTICALE ET DISPOSITIF D'EXAMEN DE L' IL LE COMPORTANT

(30) Priority: 10.12.2020 KR 20200172164
(43) Date of publication of application: 13.07.2022
(73) Proprietor: HUVITZ CO., LTD, Anyang-si, Gyeonggo-do 14055 (KR)
(72) Inventor: LEE, Jae Hyuk, 14055 Anyang-si, Gyeonggi-do (KR)
(74) Representative: IPAZ

(56) References cited:
- CN-A- 109 512 381
- KR-A- 20160 141 440
- US-A1- 2018 271 644

## Description

### CROSS-REFERENCE TO RELATED APPLICATION

This application claims benefit of priority to Korean Patent Application No. 10-2020-0172164 filed on December 10, 2020.

### FIELD OF THE DISCLOSURE

The present invention relates to an eye-examining device, and more particularly, to a device capable of horizontally and vertically rotating a heavy eye-examining instrument.

### BACKGROUND

An eye-examining device is a device that measures the visual acuity of a subject or examines the condition of the eyes, and includes various eye-examining instruments, such as an optical system for examining or observing an eye to be examined, an illumination device for illuminating the eye to be examined, and an imaging device for photographing the eye to be examined. Further, in order to examine an eye, it is necessary to examine and observe the eye to be examined in various directions while changing the positions of the eye-examining instruments described above. Accordingly, various driving devices for horizontally or vertically translating or rotating eye-examining instruments around the eye to be examined are used. However, the driving devices used in conventional eye-examining devices have drawbacks that they rotate horizontally or vertically only at a fixed location, thereby resulting in a limited driving area, or the linear or rotational driving structure is complicated and takes up a lot of space.

### Prior Art Literature

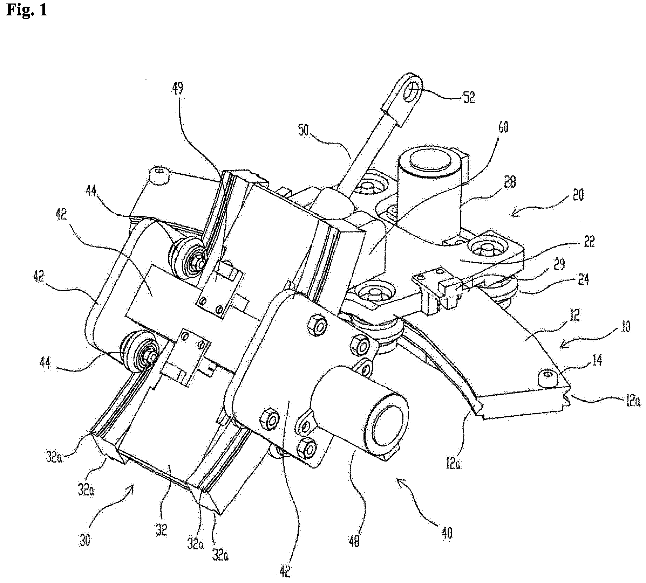

CN109 512 381 A discloses a three-dimensional motion positioning device and optometry device. In CN109 512 381, the optometry device moves in a horizontal straight line direction and a vertical straight line direction (See Figs. 1 to 6). Thus, the pan (horizontal movement) and tilt (vertical movement) structure is large in size. The centre of gravity or the driving centre of the optometry device is different from the centre of gravity of the device. Specifically, in CN109 512 381, since the optometry device moves in a horizontal straight line direction, according to the positions of the optometry device, the centre of gravity of the device changes more largely compared to that of the present invention. In addition, CN109 512 381 does not disclose the damping lever (50) and the support (80) for changing the direction of the eye examining instrument (70) which moves along the curved trajectory of the vertical curved guiding rail (32) of the present invention.
(Patent Document 1) Patent Publication No. 10-2013-0117970
(Patent Document 2) Patent Registration No. 10-1596780
(Patent Document 3) Patent Publication No. 10-2020-0097456

### SUMMARY OF THE INVENTION

### TECHNICAL OBJECTS

It is an object of the present invention to provide an eye-examining device, which has a wide horizontal and vertical rotation driving area, in which the horizontal and vertical rotation driving is performed at the center of gravity of the equipment, and which thus can be stably driven.

It is another object of the present invention to provide an eye-examining device, which can be manufactured in a small size and is driven electrically.

It is yet another object of the present invention to provide an eye-examining device, which has a wide horizontal and vertical rotation driving area and thus can easily move an eye-examining instrument to a desired position.

### TECHNICAL SOLUTION

In order to achieve the objects above, the present invention provides an eye-examining device as defined in claim 1.

### EFFECTS OF THE INVENTION

The eye-examining device in accordance with the present invention can be driven stably as the horizontal and vertical rotation driving is made at the center of gravity of the equipment. In addition, the eye-examining device in accordance with the present invention can be manufactured in a small size, are electrically driven and thus are excellent in the convenience of use, and have a wide horizontal and vertical rotation driving area.

### BRIEF DESCRIPTION OF THE DRAWINGS

Figs. 1 to 3 are respectively a perspective view, a bottom perspective view, and an exploded perspective view of a horizontal and vertical rotation driving device; and
Fig. 4 is a view for illustrating the structure and operation of an eye-examining device having a horizontal and vertical rotation driving device.

### DETAILED DESCRIPTION OF THE PREFERRED EMBODIMENTS

Hereinafter, the present invention will be described in detail with reference to the accompanying drawings. Being vertical and horizontal in this specification generally means being vertical and horizontal with respect to the ground, but comprehensively represents two different directions, including the case of being substantially vertical and horizontal with respect to the ground. Figs. 1 to 3 are respectively a perspective view, a bottom perspective view, and an exploded perspective view of a horizontal and vertical rotation driving device. As shown in Figs. 1 to 3, the horizontal and vertical rotation driving device includes a horizontal guider 10, a horizontal driving unit 20, a vertical guider 30, and a vertical driving unit 40.

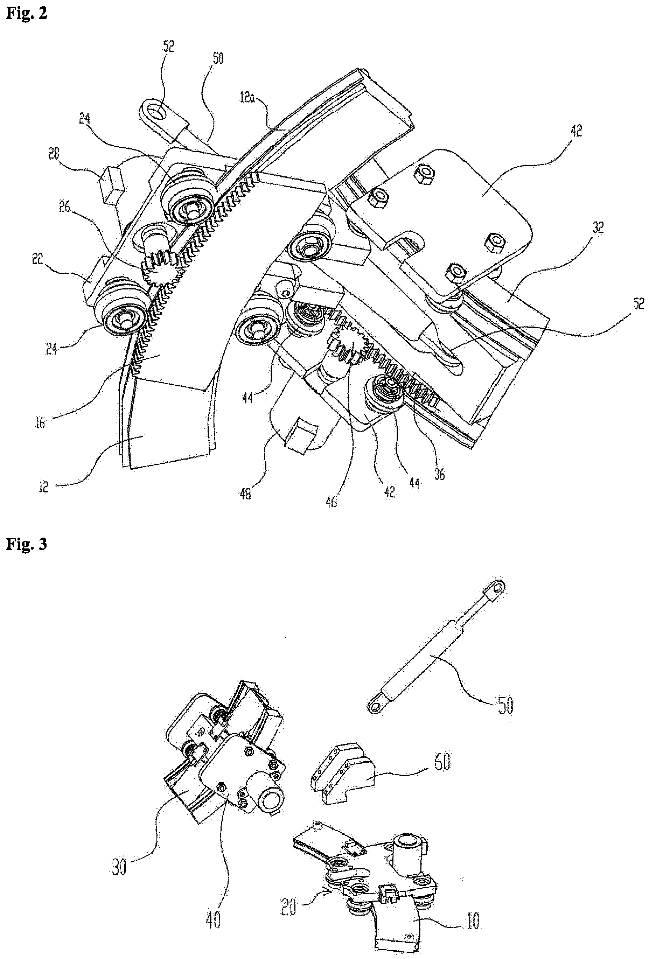

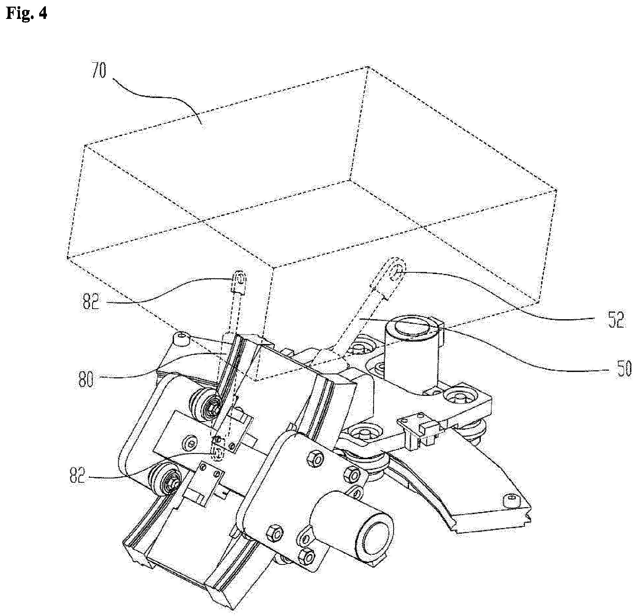

The horizontal guider 10 includes a horizontal guiding rail 12 extending by a predetermined length in the horizontal direction. The horizontal guiding rail 12 is formed along the horizontal movement path of an eye-examining instrument 70 (see Fig. 4), the horizontal driving unit 20 is mounted on the horizontal guiding rail 12, and the horizontal driving unit 20 moves in the horizontal direction along the horizontal guiding rail 12. The horizontal guiding rail 12 has a curved shape with a predetermined curvature along the horizontal movement path of the eye-examining instrument 70. Guiding grooves 12a, to which the horizontal driving unit 20 is slidably fitted so that the horizontal driving unit 20 can be guided and moved stably and that the horizontal driving unit 20 is prevented from being separated from the horizontal guiding rail 12, may be formed at one end of the horizontal guiding rail 12, for example, on both sides thereof. In addition, both ends of the horizontal guiding rail 12 may be equipped with stoppers 14 for limiting the horizontal movement area of the horizontal driving unit 20 and thereby preventing the horizontal driving unit 20 from coming off the horizontal guiding rail 12 to the outside. As shown in Fig. 2, a first gear 16 may be mounted on one end, preferably the bottom surface, of the horizontal guiding rail 12 along the path of the horizontal guiding rail 12.

The horizontal driving unit 20 is mounted on the horizontal guiding rail 12 and moves along the horizontal guiding rail 12. The horizontal driving unit 20 includes a horizontal driving body 22 to which the eye-examining instrument 70, the vertical guider 30, and the vertical driving unit 40 are coupled, and a sliding engagement portion 24 mounted on the horizontal driving body 22 and slidably coupled to the horizontal guiding rail 12. The sliding engagement portion 24 may be rail wheels that are rotatably mounted on the horizontal driving body 22 and are fitted into the guiding grooves 12a formed on both sides of the horizontal guiding rail 12 to slidingly move. If one or more, preferably two or more rail wheels are mounted on each guiding groove 12a, the horizontal driving unit 20 can move stably, which is desirable. In addition, the horizontal driving unit 20 may further include a second gear 26 that is mounted on the horizontal driving body 22, is driven by a driving unit 28 such as a motor, and meshes with the first gear 16 mounted on the horizontal guiding rail 12. Since the second gear 26 is meshed with the fixed first gear 16, when the driving unit 28 operates to rotate the second gear 26, the second gear 26 and the horizontal driving body 22 coupled thereto move along the first gear 16. Accordingly, as the driving unit 28 is rotated forward or backward, the horizontal driving unit 20 moves along the horizontal guiding rail 12. In the example shown in Fig. 2, the second gear 26 acts as a pinion gear and the first gear 16 acts as a rack gear. The horizontal driving body 22 of the horizontal driving unit 20 may be equipped with a position detection sensor 29 for detecting the position of the horizontal driving unit 20, and may detect the position of the horizontal driving unit 20 by using the position detection sensor 29, and drive the driving unit 28 accordingly, to move the horizontal driving unit 20 to a desired position.

The vertical guider 30 is fixedly coupled to the horizontal driving body 22 of the horizontal driving unit 20, moves horizontally along the horizontal driving unit 20, and guides the vertical movement of the vertical driving unit 40. As shown in Figs. 1 to 3, the vertical guider 30 and the horizontal driving body 22 may be coupled by a coupling block 60. The vertical guider 30 includes a vertical guiding rail 32 extending by a predetermined length in the vertical direction along the vertical movement path of the eye-examining instrument 70 (see Fig. 4). In a similar form as the horizontal driving unit 20, the vertical driving unit 40 is mounted on the vertical guiding rail 32 and moves in the vertical direction along the vertical guiding rail 32. The vertical guiding rail 32 has a curved shape with a predetermined curvature along the vertical movement path of the eye-examining instrument 70. Guiding grooves 32a, to which the vertical driving unit 40 is slidably fitted so that the vertical driving unit 40 can be guided and moved stably and that the vertical driving unit 40 is prevented from being separated from the vertical guiding rail 32, may be formed at one end of the vertical guiding rail 32, for example, on the front and rear surfaces thereof. In addition, both ends of the vertical guiding rail 32 may also be equipped with stoppers (not shown) for limiting the vertical movement area of the vertical driving unit 40 and thereby preventing the vertical driving unit 40 from coming off the vertical guiding rail 32 to the outside. As shown in Fig. 2, a third gear 36 may be mounted on one end, preferably the bottom surface, of the vertical guiding rail 32 along the path of the vertical guiding rail 32.

The vertical driving unit 40 is mounted on the vertical guiding rail 32 of the vertical guider 30 in a similar manner to the horizontal driving unit 20, and moves along the vertical guiding rail 32. The vertical driving unit 40 includes a vertical driving body 42 to which the eye-examining instrument 70 (see Fig. 4) is coupled, and a sliding engagement portion 44 mounted on the vertical driving body 42 and slidably coupled to the vertical guiding rail 32. The sliding engagement portion 44 may be rail wheels that are rotatably mounted to the vertical driving body 42 and that are fitted into the guiding grooves 32a formed on both sides of the vertical guiding rail 32 to slidingly move. If one or more, preferably two or more rail wheels are mounted on each guiding groove 32a, the vertical driving unit 40 can move stably, which is desirable. In addition, the vertical driving unit 40 may further include a fourth gear 46 that is mounted on the vertical driving body 42, is driven by a driving unit 48 such as a motor, and meshes with the third gear 36 mounted on the vertical guiding rail 32. Since the fourth gear 46 is meshed with the fixed third gear 36, when the driving unit 48 operates to rotate the fourth gear 46, the fourth gear 46 and the vertical driving body 42 coupled thereto move along the third gear 36. Accordingly, as the driving unit 48 is rotated forward or backward, the vertical driving unit 40 moves up and down along the vertical guiding rail 32. In the example shown in Fig. 2, the fourth gear 46 acts as a pinion gear and the third gear 36 acts as a rack gear. In the example shown in Figs. 1 to 3, although it is shown that the sliding engagement portions 44 of the vertical driving unit 40 are installed on both the left and right sides of the vertical driving body 42, respectively, and the sliding engagement portions 44 respectively installed on both the left and right sides fix the left and right sides of the vertical guiding rail 32, respectively, only one pair may be installed on one side of the horizontal guiding rail 12, similar to the sliding engagement portion 24 of the horizontal driving unit 20. The vertical driving body 42 of the vertical driving unit 40 may be equipped with a position detection sensor 49 for detecting the position of the vertical driving unit 40, and may detect the position of the vertical driving unit 40 by using the position detection sensor 49, and drive the driving unit 48 accordingly, to move the vertical driving unit 40 to a desired position.

Fig. 4 is a view for illustrating the structure and operation of an eye-examining device having a horizontal and vertical rotation driving device. As shown in Figs. 1 to 4, an eye-examining device in accordance with the present invention includes an eye-examining instrument 70 that is mounted on the horizontal and vertical rotation driving device and is driven horizontally and vertically. The eye-examining instrument 70 is coupled to the vertical driving body 42 of the vertical driving unit 40 via a support 80. Here, in order for the angle between the eye-examining instrument 70 and the vertical driving unit 40 to be changed according to the position of the vertical driving unit 40, for example, in order for the eye-examining instrument 70 to be tilted downward toward where the eye to be examined is located when the vertical driving unit 40 ascends, the support 80, the eye-examining instrument 70, and the vertical driving body 42 are pivotally coupled to each other so as to rotate about a rotation shaft 82. In addition, an eye-examining device in accordance with one embodiment of the present invention further includes a damping lever 50 (shock absorber), one end of which is coupled to the vertical guiding rail 32 and the other end is coupled to the eye-examining instrument 70, so as to support the eye-examining instrument 70, and which has a variable length, as necessary. By the damping lever 50, the eye-examining instrument 70 is supported and is coupled to the vertical guiding rail 32. By buffering and supporting one end of the eye-examining instrument 70 with the damping lever 50, the angle of the eye-examining instrument 70 can be adjusted, the user can control the eye-examining instrument 70 by applying a small force, and the eye-examining instrument 70 can be prevented from abruptly tilting or moving. Here, in order for the angle between the eye-examining instrument 70 and the vertical guider 30 to be changed according to the height of the vertical driving unit 40, it is preferable that the damping lever 50, the eye-examining instrument 70, and the vertical guider 30 are pivotally coupled to each other so as to rotate around a rotation shaft 52 (see Fig. 2).

Since the horizontal and vertical rotation driving device uses a double rail structure of the horizontal guiding rail 12 and the vertical guiding rail 32, it is possible to implement a small, decentered pan drive (horizontal movement) and tilt drive (vertical movement) structure. Conventional eye-examining devices have a drawback of unstable driving because the pan and tilt structure is large in size, and the center of gravity or the driving center of the driven device is different from the center of gravity of the eye-examining device. On the other hand, since the pan driving operates while the eye-examining instrument 70 is being separated from the eye to be examined, and the tilt driving structure is miniaturized and is at the center of gravity of the eye-examining device in the device of the present invention, there is an advantage that the structure of the eye-examining device is efficient, and horizontal and vertical rotational driving is performed stably. Further, the horizontal and vertical rotation driving device can be electrically driven by checking the positions of the horizontal driving unit 20 and the vertical driving unit 40 with the sensors 29 and 49, and can stably drive the eye-examining instrument 70 by using the damping lever 50.

Although the present invention has been described above with reference to the accompanying drawings and exemplary embodiments, the present invention is not limited to what is shown in the drawings and the embodiments described above. Reference numerals are labeled in the following claims to aid understanding, but the scope of the following claims is not limited to the reference numerals and what is shown in the drawings, and should be construed to encompass all modifications, equivalent constructions and functions of the exemplary embodiments.

## Claims

1. An eye-examining device comprising:
a horizontal guider (10) comprising a horizontal guiding rail (12) extending by a predetermined length in the horizontal direction;
a horizontal driving unit (20) that is mounted on the horizontal guiding rail (12) and moves along the horizontal guiding rail (12);
a vertical guider (30) that is fixedly coupled to the horizontal driving unit (20), moves horizontally along the horizontal driving unit (20), and comprises a vertical guiding rail (32) extending by a predetermined length in the vertical direction;
a vertical driving unit (40) that is mounted on the vertical guiding rail (32) and moves along the vertical guiding rail (32);
an eye-examining instrument (70) coupled to the vertical driving unit (40) via a support (80);
**characterized in that** it further comprises:
a damping lever (50), one end of which is coupled to the vertical guiding rail (32) and the other end is coupled to the eye-examining instrument (70), so as to support the eye-examining instrument (70), and which has a variable length;
wherein a guiding groove (32a), into which the vertical driving unit (40) is slidably fitted, is formed at one end of the vertical guiding rail (32), the vertical driving unit (40) comprises a vertical driving body (42), and a sliding engagement portion (44) mounted on the vertical driving body (42) and slidably coupled to the vertical guiding rail (32),
the support (80) is coupled to the vertical driving body (42) of the vertical driving unit (40), and the support (80), the eye-examining instrument (70), and the vertical driving body (42) are pivotally coupled to each other so as to be able to rotate about a rotation shaft (82),
the horizontal guiding rail (12) has a curved shape with a predetermined curvature along a horizontal movement path of the eye-examining instrument (70), and
the vertical guiding rail (32) has a curved shape with a predetermined curvature along a vertical movement path of the eye-examining instrument (70).

## Patentansprüche

1. Augenuntersuchungsvorrichtung, umfassend:
eine horizontale Führung (10), umfassend eine horizontale Führungsschiene (12), die sich um eine vorbestimmte Länge in horizontaler Richtung erstreckt;
eine horizontale Antriebseinheit (20), die an der horizontalen Führungsschiene (12) montiert ist und sich entlang der horizontalen Führungsschiene (12) bewegt;
eine vertikale Führung (30), die an die horizontale Antriebseinheit (20) fest gekoppelt ist, sich entlang der horizontalen Antriebseinheit (20) horizontal bewegt und eine vertikale Führungsschiene (32) umfasst, die sich um eine vorbestimmte Länge in vertikaler Richtung erstreckt;
eine vertikale Antriebseinheit (40), die an der vertikalen Führungsschiene (32) montiert ist und sich entlang der vertikalen Führungsschiene (32) bewegt;
ein Augenuntersuchungsinstrument (70), das an die vertikale Antriebseinheit (40) über eine Stütze (80) gekoppelt ist;
**dadurch gekennzeichnet, dass** sie ferner umfasst:
einen Dämpfungshebel (50), dessen eines Ende an die vertikale Führungsschiene (32) gekoppelt ist und dessen anderes Ende an das Augenuntersuchungsinstrument (70) gekoppelt ist, um das Augenuntersuchungsinstrument (70) zu stützen, und der eine variable Länge aufweist;
wobei eine Führungsnut (32a), in die die vertikale Antriebseinheit (40) gleitend eingepasst ist, an einem Ende der vertikalen Führungsschiene (32) ausgebildet ist, die vertikale Antriebseinheit (40) einen vertikalen Antriebskörper (42) und einen Gleiteingriffsabschnitt (44) umfasst, der an dem vertikalen Antriebskörper (42) montiert und an die vertikale Führungsschiene (32) gleitend gekoppelt ist,
die Stütze (80) an den vertikalen Antriebskörper (42) der vertikalen Antriebseinheit (40) gekoppelt ist, und die Stütze (80), das Augenuntersuchungsinstrument (70) und der vertikale Antriebskörper (42) miteinander schwenkbar gekoppelt sind, um sich um eine Drehwelle (82) drehen zu können,
die horizontale Führungsschiene (12) eine gekrümmte Form mit einer vorbestimmten Krümmung entlang eines horizontalen Bewegungspfads des Augenuntersuchungsinstruments (70) aufweist, und
die vertikale Führungsschiene (32) eine gekrümmte Form mit einer vorbestimmten Krümmung entlang eines vertikalen Bewegungspfads des Augenuntersuchungsinstruments (70) aufweist.

## Revendications

1. Dispositif d'examen oculaire comprenant :
un guide horizontal (10) comprenant un rail de guidage horizontal (12) s'étendant sur une longueur prédéterminée dans la direction horizontale ;
une unité d'entraînement horizontale (20) qui est montée sur le rail de guidage horizontal (12) et se déplace le long du rail de guidage horizontal (12) ;
un guide vertical (30) qui est couplé de manière fixe à l'unité d'entraînement horizontale (20), se déplace horizontalement le long de l'unité d'entraînement horizontale (20), et comprend un rail de guidage vertical (32) s'étendant sur une longueur prédéterminée dans la direction verticale ;
une unité d'entraînement verticale (40) qui est montée sur le rail de guidage vertical (32) et se déplace le long du rail de guidage vertical (32) ;
un instrument d'examen oculaire (70) couplé à l'unité d'entraînement verticale (40) par l'intermédiaire d'un support (80) ;
**caractérisé en ce qu'**il comprend en outre :
un levier d'amortissement (50), dont une extrémité est couplée au rail de guidage vertical (32) et l'autre extrémité est couplée à l'instrument d'examen oculaire (70), de manière à supporter l'instrument d'examen oculaire (70), et qui présente une longueur variable ;
dans lequel une rainure de guidage (32a), dans laquelle l'unité d'entraînement verticale (40) est montée de manière coulissante, est formée à une extrémité du rail de guidage vertical (32), l'unité d'entraînement verticale (40) comprend un corps d'entraînement vertical (42) et une partie d'engagement coulissant (44) montée sur le corps d'entraînement vertical (42) et couplée de manière coulissante au rail de guidage vertical (32),
le support (80) est couplé au corps d'entraînement vertical (42) de l'unité d'entraînement verticale (40), et le support (80), l'instrument d'examen oculaire (70) et le corps d'entraînement vertical (42) sont couplés de manière pivotante les uns aux autres de manière à pouvoir tourner autour d'un arbre de rotation (82),
le rail de guidage horizontal (12) présente une forme courbe avec une courbure prédéterminée le long d'un trajet de déplacement horizontal de l'instrument d'examen oculaire (70), et
le rail de guidage vertical (32) présente une forme courbe avec une courbure prédéterminée le long d'un trajet de déplacement vertical de l'instrument d'examen oculaire (70).
